(19) Europäisches Patentamt
European Patent Office
Office européen des brevets

(11) EP 0 311 632 B1

(12) EUROPEAN PATENT SPECIFICATION

(45) Date of publication and mention of the grant of the patent:
**29.05.1996 Bulletin 1996/22**

(21) Application number: **87904340.4**

(22) Date of filing: **16.06.1987**

(51) Int Cl.6: **C07D 207/267**, C07D 207/27, C07D 211/76, C07D 223/10

(86) International application number:
**PCT/US87/01391**

(87) International publication number:
**WO 88/00184 (14.01.1988 Gazette 1988/02)**

# (54) SURFACE ACTIVE LACTAMS

OBERFLÄCHENAKTIVE LAKTAME

LACTAMES TENSIO-ACTIVES

(84) Designated Contracting States:
**AT BE CH DE FR GB IT LI NL**

(30) Priority: **27.06.1986 US 879776**
**12.02.1987 US 13760**

(43) Date of publication of application:
**19.04.1989 Bulletin 1989/16**

(73) Proprietor: **ISP INVESTMENTS INC.**
**Wilmington, Delaware 19801 (US)**

(72) Inventors:
- **LOGIN, Robert, B.**
  **Oakland, NJ 07436 (US)**
- **CHAUDHURI, Ratan, K.**
  **Butler, NJ 07405 (US)**
- **HASHEM, Mohamed, M.**
  **Wayne, NJ 07470 (US)**
- **HELIOFF, Michael, W.**
  **Westfield, NJ 07090 (US)**
- **PRITCHARD, David, W.**
  **Vernon, NJ 07462 (US)**
- **RUPPERT, Ronald, M.**
  **Moonachie, NJ 07074 (US)**
- **SAVIO, Lenore, E.**
  **Somerset, NJ 08873 (US)**
- **SUWALA, David, W.**
  **Dover, DE 19901 (US)**
- **TRACY, David, J.**
  **Lincoln Park, NJ 07035 (US)**

(74) Representative: **Ford, Michael Frederick et al**
**MEWBURN ELLIS**
**York House**
**23 Kingsway**
**London WC2B 6HP (GB)**

(56) References cited:
**DE-A- 2 252 102** **US-A- 3 988 318**
**US-A- 3 988 350** **US-A- 4 557 934**

- **Journ. of the Amer. Chem. Soc., Volume 80 March 1947, F.B. ZIENTY and G.W.**
- **STEAHLY, "N-Substituted 2-Pyrrolidones", pp. 715-717 (see pages 715-716).**
- **CHEM. ABSTRACTS, Vol. 80, No. 8, 41385z; issued 1974 February 24 (Columbus, Ohio, U.S.A.) MASAYUKI et al., "Phase Separation and Surface Tension of Aqueous Hydrochloric Acid Solution of N-Doodecyl-2-Pyrrolidone, p. 298, columns 1 and 2 Nippon Kagaku Kaishi, 1973, (11), 2056-62 (Japan).**
- **CHEM. ABSTRACTS, Vol. 84, No. 26, 185540b, issued 1976, June 28 (Columbus, Ohio U.S.A.) MASAYUKI et al., "The effect of Additives on the Solubility of 1- Dodecyl-2-Pyrrolidone in Aqueous Solutions", p. 357, col. 2, Nippon Kaguku Kaishi, 1976, (4), 547-53 (Japan).**
- **CHEM. ABSTRACTS, Vol. 104, No. 10, 74916r, issued 1986, March 10 (Columbus, Ohio, USA) OHSHIMA et al., "Enhancing Effect of Absorption Promoters... Study on the Absorption Promoting Effect of Azone", p. 385, col. 1, J. Pharmacobio- Dyn., 1985, 8(11), 900-5(Eng).**

EP 0 311 632 B1

Printed by Jouve, 75001 PARIS (FR)

- CHEM. ABSTRACTS, Vol. 105, No. 18, 158716j, issued 1986, November 3 (Columbus, Ohio, USA) MARAKAMI et al., "Promoting Effect of Azone on Intestinal Absorption of Poorly Absorbable Drugs in Rats", p. 366, col. 2, Int. J. Pharm., 1986, 31(3), 231-8(Eng.)
- 31(3), 231-8(Eng.)
- CHEM. ABSTRACTS, Vol. 87, No. 23, 184365y, issued 1977, December 5 (Columbus, Ohio, USA) SIMAK et al., "Iodine Complexes of Dialkyl Acid Amides and N-Alkyl Lactams", p. 598, col. 2, Ger Offen. 2611602 (C1.C07D 207/26) 22 Sep. 1977, 14 pp.
- CHEM. ABSTRACTS, Vol. 91, NO. 23, 192602w; issued 1979, December 3 (Columbus, Ohio USA) RUOSTESUO, "Studies on the Polarity of 1-Phenyl-2-Pyrrolidinones, Sulfonamides and Sulfenamides", p.582, columns 1 and 2, Acta Univ. Ouluensis, Ser. A 1978, 66, 53 pp. (Eng.)
- CHEM. ABSTRACTS, Vol. 95, No. 26, 230648h issued 1981, December 28 (Columbus, Ohio USA) Jpn. Tokkoyo Koho 81 35,018 (C1. H01G9/02, 14 Aug. 1981, Nichicon Capacitor Ltd., 5 pp.
- CHEM. ABSTRACTS, Vol. 90, No. 25, 203295f, issued 1979, June 18 (Columbus, Ohio, USA) KARJALAINEN, et al.. "Hydrogen Bonding of Pyrrole, Indole and Carbazole with Substituded 1-Phenyl-2-Pyrrolidinones", p.544, columns 1 and 2, Acta Chem. Scanda., Ser. a 1979, A33(1), 76-8 (Eng.)
- CHEM. ABSTRACTS, Vol. 93, No. 25, 232708k, issued 1980, December 22 (Columbus, Ohio USA) McCARTHY et al. "Mixtures or Addition Compounds of Haloalkyl Phosphonic Acids and Amides or Amide Polymers, and their use as Plant Growth Regulants", p.247, col. 1, Brit. Uk Pat. Appl. 2029832 (C1.C07F9/28), 26 Mar. 1980, US Appl. 935945, 23 Aug. 1978; 24 pp.
- CHEM. ABSTRACTS, Vol. 95, No. 21, 187417t issued 1981, November 23, (Columbus, Ohio USA) McCARTHY et al (Gaf Corp.) Fr. Demande 2447930 (C1. C07F9/65), 29 Aug. 1980, US Appl. 7769, 30 Jan. 1979; Phosphoranyl Derivatives Containing Positively Charged Nitrogen", 36 pp.
- CHEM. ABSTRACTS, Vol. 78, No. 1 75p. issued 1973, January 8 (Columbus, Ohio, USA) YOSHIHARA et al., "Metabolism of Drugs. LXXVI. Metabolic Fate of Prolintane in Rabbits" p.83, col. 1, Chem. Pharm. Bull. 1972, 20(9), 1906-12 (Eng.)
- CHEM. ABSTRACTS, Vol. 84, No. 19, 134762t, issued 1976, May 10 Columbus, Ohio, USA) VIRTANEN et al., "The Association of Substituded 1-Phenyl-2-Pyrrolidones with Phenol", p. 425, col. 1, Finn. Chem. Lett. 1975, (7-8), 183-6 (Eng.)
- CHEM. ABSTRACTS, Vol. 98, No. 15, 125489w, issued 1983, April 11 (Columbus, Ohio, USA) YAMAMOTO et al."Ullman Condensation Using Copper or Copper Oxide as the Reactant. Arylation of Active Hydrogen Compounds (Imides, amides, amines, Phenol, Benzoic Acid and Phenylacetylene", p.586, col. 1, Can. J. Chem. 1983 61(1); 86-91 (Eng)
- Ohio, USA) YAMAMOTO et al." "Ullman Condensation Using Copper or Copper Oxide as the Reactant. Arylation of Active Hydrogen Compounds (Imides, Amides, Amines, Phenol, Benzoic Acid and Phenylacetylene", p.586, col. 1, Can. J. Chem. 1983 61(1), 86-91 (Eng.).


Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to the utilization of a particular group of N-hydrocarbon substituted lactams having surfactant properties.

N-lower alkyl pyrrolidones have found wide commercial acceptance as non-toxic aprotic chemical solvents. However, absence of hydrophobic-lipophobic balance in these molecules, as in the case of N-methyl pyrrolidone, prevents micellular formation; consequently they possess no significant aqueous surfactant properties. N- higher alkyl pyrrolidones have been used as adjuvants in pesticidal compositions: EP-A-77 078 and WPI 75-50048 W.

According to a first aspect of this invention, there is provided a composition comprising

(i) a N-hydrocarbon substituted lactam surfactant having the formula

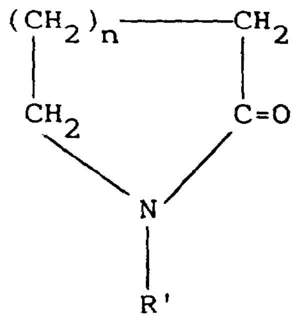

wherein n is an integer having a value of from 1 to 3 and R' is a hydrophobic radical consisting of a linear, branched chain or cyclic alkyl radical containing from 7 to 22 carbon atoms; a naphthyl or alkyl substituted naphthyl radical containing from 10 to 26 carbon atoms or an alkylphenyl or phenylalkyl radical containing from 9 to 26 carbon atoms; which lactams are capable of forming micelles in neutral, basic or acidic aqueous media or have a critical micelle concentration of between about 1 x $10^{-3}$ and about 5 x $10^{-5}$ moles per liter, and

(ii) at least one water-insoluble substance other than a said lactam surfactant, and is selected from insecticides, herbicides, fungicides, pesticides, plant growth regulators and annelidicides;

which composition is in the form of a liquid concentrate or a wettable powder and forms an emulsion or suspension of the substance upon dilution with water.

Of the above lactam surfactants, the N-alkyl lactams are preferred and, of these, N-alkyl pyrrolidones containing 8 to 18 carbon atoms and having a critical micelle concentration less than 2 x $10^{-2}$ moles per liter are most preferred.

While it is intended to include lactams substituted with cycloalkyl R' groups, it is found that the hydrophobic affect of these cyclic groups is less than that of their linear analogs. Accordingly, where a N-cycloalkyl R' group is intended, the R' moiety preferably contains at least 8 carbon atoms to provide hydrophobic-lipophobic balance.

The cyclic hydrophilic moiety of the compounds herein defined is an important factor in maximizing their efficiency by concentrating the molecule, and thus the surfactant activity, at the interface and minimizing solubility of the molecule in liquid phases.

The above properties demonstrate the high efficiency and activity of the present nonionic surfactants. Such surfactant properties are unexpected since the compounds lack the polyalkoxy groups commonly associated with conventional nonionic surfactants. By way of comparison, N-dodecyl-2-pyrrolidone, exhibits a surfactant properties equivalent to a lauryl alcohol containing five moles of ethoxylate, i.e. $C_{12}H_{25}$-$(CH_2CH_2O)_5H$, a known commercial nonionic surfactant.

Not all of the present lactams possess good aqueous surfactant properties. For example, the pyrrolidones having R' substituents containing less than 8 carbon atoms do not have hydrophobic groups of a length sufficient to form stable micelles in water. On the other hand, R' alkyl substituents of hexadecyl and above exhibit an imbalance such that the hydrophilic pyrrolidone or caprolactam moiety cannot counteract the dominating hydrophobic character of the alkyl group. However, these higher molecular weight compounds can provide surfactant properties in non-aqueous systems and are valuable wetting agents in applications involving solid substrates.

The structure of the present lactams provides a key to their unique properties. Specifically, the highly polar and hydrophilic pyrrolidone moiety exhibits several resonance forms, i.e.

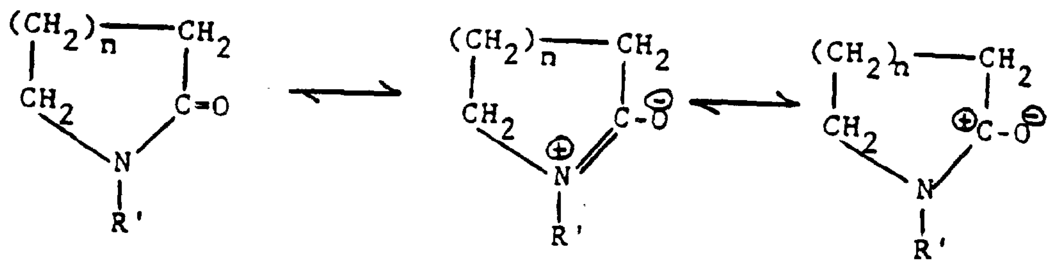

B.

The existence of such multiple resonance states contributes to the high dipole moment of about 4 debye, possessed by these lactams. A high dipole moment is an important property, conferring the ability of these molecules to assume multiple resonance forms which promotes their tendency to complex or interact with many chemical groups. For example, the ability of these compounds to interact with anionic moieties, e.g. sulfate groups, is beneficial in enhancing foam and reducing skin irritability of various medicinal creams and lotions containing such or similar anionic groups.

The above lactam products having a molecular weight of from about 180 to about 450 are conveniently prepared by several known processes including the reaction between a lactone having the formula

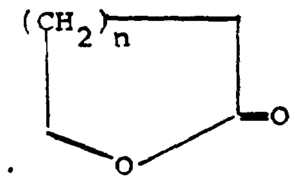

C.

wherein n is as defined above, and an amine having the formula R'-$NH_2$ wherein R' is as defined above. The amine reactant having the formula R'-$NH_2$ includes alkylamines having from 7 to 20 carbon atoms; naphthyl or alkylnapthyl amines having from 10 to 26 carbon atoms; alkylphenyl or phenylalkyl amines having from 9 to 26 carbon atoms; amines derived from natural products, such as coconut amines or tallow amines and distilled cuts or hydrogenated derivatives of such fatty amines. Also, mixtures of amine reactants can be used in the process for preparing the present compounds. Such mixtures can include cyclic, linear and branched chain amino species having an alkyl or other organic substituent of the same or different molecular weight. In the present process the amine and lactone reactants, combined in a mole ratio of from about 1:1 to about 1:5, are reacted under conditions of constant agitation, at a temperature between about 200°C. and about 350°C. under a pressure of from atmospheric to about 650 psig for a period of from about 1 to about 15 hours; preferably at 250°C. to 300°C. under an initial ambient pressure for a period of from 5 to 10 hours. The resulting lactam product is recovered and purified by distillation or by any other convenient recovery process.

The N-alkyl lactam products having 11 to 14 carbon atoms are clear, water white liquids, at room temperature; whereas those having 16 or more carbon atoms are solids. These lactams have a neutral or slightly basic pH, a surface tension between about 25 and about 35 dynes/cm as a 0.1% water solution and a viscosity of from about 6 to about 30 cps at 25°C. More particularly, these lactams of 97% or higher purity are capable of producing micelles at a critical molar micelle concentration less than 1 x $10^{-3}$. For the N-alkyl-2- pyrrolidones species the capability of producing micelles at a critical micelle concentration of between about 4.4 x $10^{-4}$ and about 5 x $10^{-5}$ is obtainable. Generally, the $C_8$ to $C_{14}$ alkyl lactams display primarily surfactant properties; whereas the $C_{16}$ to $C_{22}$ alkyl species are primarily complexing agents; although some degree of surfactants and complexing capability exists in all of the present species.

The maximum surface concentration of the present surfactants at equilibrum above the critical micelle concentration is exceptionally high, i.e. between about 2 moles/cm x $10^{-10}$ and about 5 moles/cm x $10^{-10}$. It is believed that the monodisperse nature of the hydrophobe and hydrophile moieties of the these surfactants contribute to the high surface concentrations by maximizing entropy of packing, particularly in the case of the N-decyl-2- pyrrolidone species which exhibits essentially equal hydrophobe/hydrophobe and hydrophile/hydrophile interactions at the liquid interface, while longer R groups reduce packing due to steric effects.

The present group of lactams also exhibit a unique and heretofore unrealized combination of properties, suitable for increasing rates of solubilization for solids in a liquid medium in which they are normally insoluble.

A particularly beneficial property of instant lactams is their complexing ability in which the complexed active compound is positioned on the micelle surface. The lactam containing micelles in turn migrate to the surface of a bulk solution forming a monomolecular film thereon; thus concentrating the chemical which has been complexed at a solution-substrate interface. This spacial concentration of the active ingredient at the liquid surface can provide more efficient contact between the complexed chemical and a substrate, thus permitting the efficient use of an active ingredient to perform its intended function. The lower concentration of an active ingredient can result in lowering toxicity and alleviating undesirable side effects.

Still another benefit obtained by the complexing capability of the present compounds is realized by their ability to reduce toxicity of those systemic and non-systemic chemicals whose toxicity levels exceed the use intended. Pesticides, insecticides, fungicides and herbicides all contain members in this category. For example, 2-methyl-2-(methythio) propionaldehyde O-(methyl carbamoyl) oxime, known as Aldicarb, when mixed with water produces a ecologically hazardous mixture. However, complexing this compound with one of the present compounds reduces its toxicity to a level sufficient to effectively kill household insects without danger to humans. The toxicity of phenyl mercuric compounds such as the acetate, borate, chloride, hydroxide, nitrate, naphthenate, oleate, propionate and salicylate as well as that of aldrin and dieldrin are significantly reduced through complexing. Other herbicides and pesticides having normal required toxicity can be incorporated with the lactams of this invention to provide better adhesion on the surface of plant tissue, increased persistence of chemical action, reduced interfacial tension between the active component and plant surface and other benefits commensurate with the particular properties of the lactam selected. Also, because the present compounds tend to concentrate the active ingredient at the surface of its micellular structure, smaller dosages of the active ingredient can be employed and more efficiently utilized.

The performance of non-systemic active ingredients depends on the quality of the spray deposit. Particularly fungicides require a complete and uniform coverage which is resistant to heavy dews, rainfall and sprinkler irrigation. N-n-dodecyl-2-propylene promoted polyvinylpyrrolidone, most preferably in about an 80/20 wt % mixture with an alkyd-resin based compound is particularly beneficial in providing good sticking performance on crops with a wide variety of active ingredients. Application levels of from about 30 to about 130 ml/100 liters of spray, depending on total spray volume, type of crop and active ingredient, are suitable.

Also, superior oil/surfactant blends are formed, e.g. by 85/15 wt % mixtures of N-octyl-2-pyrrolidone and N-dodecyl-2-pyrrolidone, with a non-phytotoxic paraffin oil. Such blends are more effective than conventional types of spray oils because of the synergestic effect between the spray oil and the surfactant blend.

Another remarkable and unexpected property of the N-alkyl pyrrolidones of this invention is the reverse solubility exhibited by the $C_8$ and $C_{14}$ alkyl species, hereinafter discussed in more detail.

The cloud points of 10% aqueous solutions of the following compounds are reported for various N-alkyl-2-pyrrolidones

| alkyl group | Cloud Point |
|---|---|
| cyclohexyl | 55°C. |
| octyl | < 0°C. |
| decyl | 19°C. |
| dodecyl | 15-19°C. |
| tetradecyl | 33°C. |
| coco | 22-23°C. |
| hexadecyl | insoluble |

The cloud points indicate the ability of the lactam molecule to retain water of hydration. The higher the cloud point, the more soluble, i.e. hydrated, the surfactant. As shown above, the cyclohexyl pyrrolidone species is highly soluble, requiring 55°C. to effect dehydration. However, this species fails to form stable micelles. Conversely, the octyl pyrrolidone species has a cloud point below room temperature and is almost insoluble. It appears that the octyl group has insufficient length to counteract the action of the pyrrolidone groups in the interactions (a) and (b)

(a) 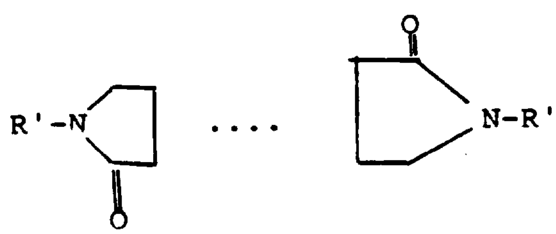

vs

D.

(b) 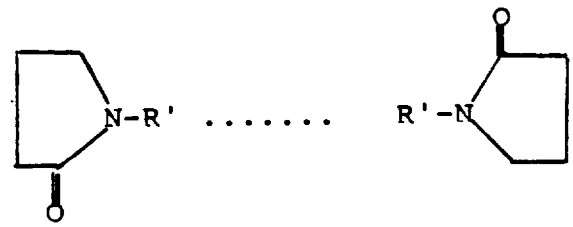

For the octyl species, it is believed that interaction (a) predominates and the resulting dimer is insoluble for the reason that the R' groups are exposed in terminal positions of the interacted molecules thus reducing the hydration of the pyrrolidone moieties. Conversely, for longer chain R' groups, interaction (b) predominates and stable micelles i.e.

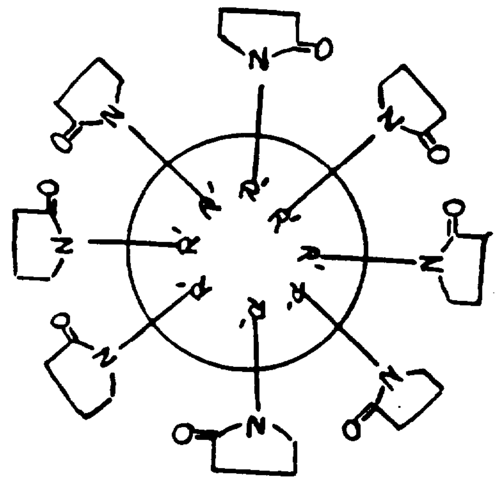

E.

where interaction occurs among the R' groups, are then formed.

The above properties combine so that lactams as defined above are useful (1) in the solubilization of insoluble compounds such as agricultural chemicals to form surface active compositions, (2) as surfactants for wettable powders and (3) as detoxifying agents.

The lactams of this invention are soluble in most organic solvents including glycerin, acetone, lower molecular weight alcohols, toluene, polyethylene glycols, polypropylene glycols, xylene, heptane, methylene chloride, perhalogenated lower molecular weight hydrocarbons, paraffin oil, Stoddard solvent, etc., and are compatible with all classes of surfactants under varying pH conditions. The water dispersibility properties of the $C_{10}$ to $C_{14}$ alkyl species seems to contravert the generally accepted chemical behaviour of hydrocarbon compounds. One would expect that as the alkyl chain length increases, the compound would become more hydrophobic. Instead, it is now discovered that while N-octyl pyrrolidone is substantially insoluble in water, the N-decyl species shows partial solubility and the N-dodecyl and N-tetradecyl pyrrolidones are completely water dispersable. Water insolubility is again evidenced in the pyrrolidones having alkyl group of 16 or more carbon atoms, for the reasons explained above. However, blends of higher alkyl species with the $C_8$ to $C_{14}$ alkyl species are water dispersable by algebraic summation of the individual contributions of the respective alkyl and pyrrolidone moieties to the hydrophobic/hydrophilic balance. Also a hydrotrope or a mineral acid electrolyte e.g., a hydrogen halide such as HC1, sulfuric acid, phosphoric acid, xylene sulfonate, toluene sulfonate, cumene sulfonate, an alcohol, a short chain fatty acid, in low level concentration, aids in improving water dispersibility of the N-$C_7$ to $C_{10}$ alkyl pyrrolidones since they are readily solubilized in aqueous solutions of pH 6 or less.

The excellent wetting, fixative and complexing properties of the $C_7$ to $C_{20}$ alkyl-2-pyrrolidones are beneficial in formulations for insecticides, herbicides, plant growth regulants, pesticides, annelidicides, etc. as a fixing agent to retain the active agent on the surface of the plant membrane.

The excellent wetting properties of instant lactams recommends them as outstanding surfactants for use in concentrate formulations of wettable powders, particularly fungicidal and herbicidal powders, which are subsequently diluted with water to form aqueous suspensions suitably employed as sprays for application on crops. These suspensions which include the lactam in the active concentrate formulation are distinguished by significantly improved stability over extended periods. The method for preparing the concentrate involves blending the active component, preferably having a particle size less than 25 micrometers, with an inert carrier, a dispersing agent to prevent flocculation and the lactam wetting agent to facilitate the suspension of particles. The blend is generally milled, eg. on a fly cutter mill operated at about 20,000 rpm, at ambient temperature for a period of from about 0.5 to about 5 minutes. This operation provides a wettable powder concentrate having a suspensibility greater than 75% and a wetting time of less than 3 seconds. Suitable fillers for the concentrate include clays, talc, silica powder, bentonite, and diatomaceous earth. The blends of

anionic dispersing agent and the nonionic lactam with the wettable powder and filler ensures good storage stability and improves suspension properties upon dilution in the field. The present lactams are highly compatible with anionic dispersants commonly employed, such as Igepon T-77 (a sodium salt of fatty acid amide sulfonate), Blancol (the sodium salt of sulfonated naphthalene-formaldehyde condensate) and Marasperse N (a lignosulfonate). The lactams also guarantee good formulation and tank mixing capability with the most common active ingredients.

The present lactams are also useful in the preparation of emulsifiable concentrates of agricultural chemicals which, when added to water, form a sprayable oil-in-water emulsion having dispersed phase droplets in the range of from about 0.1 to about 5 micrometers. Such an emulsion provides a uniform and accurate application of the active ingredient, eg. on the crops, and ensures uniform spreading and wetting under normal spray and weather conditions to form such emulsifiable concentrates. The lactams of this invention can be combined with at least one other amphoteric, anionic, non-ionic or cationic surfactant in a weight ratio of between about 1:10 and about 1:0.8 preferably between about 1:9 and about 1:1 at 15-30°C. Suitable co-surfactants include EMULPHOR® EL-620 (polyethoxylated av. 30 castor oil); EMULPHOR® EL-719 (polyethoxylated av. 40 castor oil); IGEPAL® CO-630 (ethoxylated av. 9 nonylphenol); IGEPAL® CO-530 (ethoxylated av. 6 nonylphenol); KATANOL® L-2 (trichlorobenzene), MIRANOL® DN (a stearoamphoacetate); SPANS 40 (sorbitan monopalmitate), ANTARON® (a carboxyl cocoimidazoline), FENOPON® (coconut or myristic acid ester of sodium isethionate) and alkylamine guanidine polyoxyethanol.

Such emulsifiable concentrates are particularly useful in the preparation of herbicidal, fungicidal and insecticidal stable or fast breaking emulsions.

Consequently, in a second aspect, this invention provides a process of applying a water-insoluble substance to land or crops comprising the steps of diluting a composition containing the substance with water, to form an aqueous emulsion or suspension of the substance, and then spraying the emulsion or suspension onto the crops or land, the composition comprising a lactam surfactant as defined initially and at least one water-insoluble substance which is other than a said lactam surfactant.

In yet another aspect this invention provides the use of a lactam surfactant as defined initially to form a concentrate containing at least one water-insoluble substance, which concentrate is suitable for dilution with water to yield an aqueous suspension or emulsion of the substance for spraying onto crops or land.

In view of the diverse fields of application in which the present compounds are beneficially employed, it will be appreciated that widely varying amounts of these compounds can be used to fulfill their requirements and other functions which may become obvious. Generally, the amount employed is within the mole ratio range of between about 0.5:1 and about 99:1 lactam to active component and an amount at least sufficient to retain the beneficial characteristics of the lactam but not more than the amount needed to preserve the effect of the active component being complexed.

Having thus described the invention, reference is now had to the following examples which set forth preferred embodiments but which are not to be construed as limiting to the scope of the invention. It should be recognized that these examples are presented only to more specifically describe the invention, to exemplify preferred embodiments and to provide representative examples of use from which other uses and applications will become apparent.

## EXAMPLE I

Into a stainless steel autoclave was introduced n-octylamine (2342 g) and butyrolactone (1704 g) in a mole ratio of about 1:1.1. The autoclave was sealed and 100 psig of nitrogen applied and the contents heated to 275°C. and held for 8 hours during which time the reaction mixture was agitated and the pressure increased from atmospheric to about 480 psig. The reaction produced a liquid product which was recovered from the autoclave and distilled to produce 98.5% pure clear water white liquid N-octyl-2-pyrrolidone in a yield of 98% and having a boiling point of 118°C. at 0.5 mm of Hg, a viscosity of 8 cps, and a pH of 6.5 (10% in 50/50 isopropyl alcohol-water).

## EXAMPLE II

The procedure described in Example I was repeated except that decylamine was substituted for n-octylamine and the reaction product was distilled to provide 99.3% pure clear, water white liquid N-decyl-2-pyrrolidone in a yield of 98% and having a boiling point of 120°C. at 0.2 mm of Hg, a viscosity of 12 cps, and a pH (10% in 50/50 isopropyl alcohol-water) of 5.4.

## EXAMPLE III

The procedure described in Example I was repeated except that dodecylamine (Armeen 12D) was substituted for n-octylamine and the reaction product was distilled to provide 99.2% pure clear, water white liquid N-dodecyl-2-pyrrolidone in a yield of 99% and having a boiling point of 145°C. at 0.2 mm of Hg, a viscosity of 17 cps, a cloud point (10% $H_2O$ soln.) of 15-19°C., and a pH (10% in 50/50 isopropyl alcohol-water) of 7.3.

EXAMPLE IV

The procedure described in Example I was repeated except that tetradecylamine was substituted for n-octylamine and the reaction product was distilled to provide 96.9% pure clear, water white liquid N-tetradecyl-2-pyrrolidone in a yield of 95% and having a boiling point of 190°C. at 1.0 mm of Hg, a viscosity of 2 cps, a cloud point (10% $H_2O$ soln.) of 33°C., and a pH (10% in 50/50 isopropyl alcohol-water) of 5.9.

EXAMPLE V

The procedure described in Example I was repeated except that hexadecylamine (Armeen 16D) was substituted for n-octylamine and the reaction product was distilled using a hot water condenser and was discharged at about 70°C. to provide 94.5% pure N-hexadecyl-2-pyrrolidone solid in a yield of 90% and having a boiling point of 180°C. at 0.1 mm Hg.

EXAMPLE VI

The procedure described in Example I was repeated except that octadecylamine (Armeen 18D) was substituted for n-octylamine and the product was stripped of water and excess butyrolactone and was discharged at about 75°C. to provide 96% pure N-octadecyl-2-pyrrolidone solid in a yield of 96%.

EXAMPLE VII

The procedure described in Example I is repeated except that eicosylamine is substituted for octylamine and the product distilled using a warm water condenser and was discharged at about 85°C. to provide 95% pure N-eicosyl-2-pyrrolidone solid in a yield of about 90%.

EXAMPLE VIII

The procedure of Example I was repeated except that 2-ethylhexylamine was substituted for n-octylamine. The reaction product was distilled to provide 99.5% of pure clear water white liquid N-2-ethylhexylpyrrolidone-2, boiling at 92-97°C. at 0.06 mm Hg in 96% yield.

EXAMPLE IX

The procedure of Example I was repeated except that Primene 81R (a tertiary $C_{12}$-$C_{14}$ alkyl primary amine) was substituted for n-octylamine. The reaction product was distilled to provide 97% pure liquid alkylpyrrolidone product in 40% yield. This product boils at 112-115°C. at 0.7 mm Hg.

EXAMPLE X

The procedure of Example I was repeated except that t-octyl primary amine was substituted for n-octylamine. The reaction product was distilled at 98-102°C. and 0.5 mm Hg to provide 99.3% pure product in 50% yield.

EXAMPLE XI

The procedure of Example I was repeated except that α-naphthyl amine is substituted for n-octylamine. The reaction in this case yields a solid product which is recovered from the autoclave and distilled to provide 99% pure product in 50% yield. Product has a melting point of 110-112°C.

The same procedure is employed to produce N-alkyl naphthyl lactams by substituting the appropriate N-alkyl naphthyl amine for naphthyl amine in this example.

EXAMPLE XII

The procedure of Example I was repeated except that aniline is substituted for n-octylamine. The product is recovered from the autoclave and distilled at 123°C. under 0.2 mm Hg to provide 99% pure product in 50% yield.

The same procedure is employed to produce N-alkylaniline lactams by substituting the appropriate N-alkylaniline for aniline in this example.

EXAMPLE XIII

The procedure of Example I was repeated except that cocoamine distillate was substituted for n-octylamine. The reaction product was stripped of water and excess butyrolactone and discharged. The product was obtained in 96% purity and 97% yield.

EXAMPLE XIV

The procedure of Example I is repeated except that caprolactone is substituted for butyrolactone and hexadecyl amine is substituted for n-octylamine. The product,

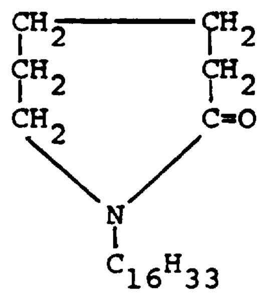

is recovered in 90% yield and purity.

EXAMPLE XV

Preparation of N-n-decyl-2-pyrrolidone

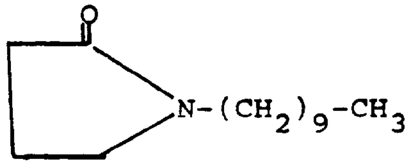

In a glass reactor, 19 g (0.22 M) of γ-butyrolactone and 34.6 g (0.22 M) of n-decylamine are mixed and heated to 180° in a round bottom flask equipped with a condenser and a Dean-Stark trap for 22 hours. The dark brown reaction mixture is distilled at reduced pressure to yield 50 g (82.5%) of colorless product; b.p. 150°-155°/0.5-1 mm Hg.

EXAMPLE XVI

Preparation of N-n-octyl-2-caprolactam having the formula

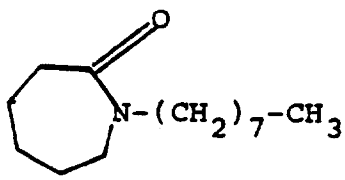

Following Example XV, heating 17.5 g (0.153 M) of 6-hexanolactone and 22 g (0.17 M) of 1-aminooctane at 180° for 29 hours gives 9 g (27%) of product; b.p. 155°-160°/0.5 mm Hg.

EXAMPLE XVII

Preparation of N-n-nonylcaprolactam having the formula

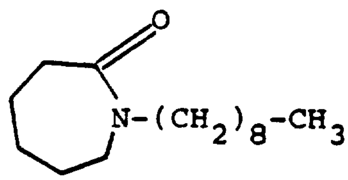

Following Example XV, heating 23 g (0.2 M) of 6-hexanolactone and 28.65 g (0.2 M) of 1-aminononane at 180° for 20 hours gives 11.5 g (26%) of product; b.p. 155°-165°/0.6 mm Hg.

EXAMPLE XVIII

Preparation of N-n-heptyl-2-caprolactam having the formula

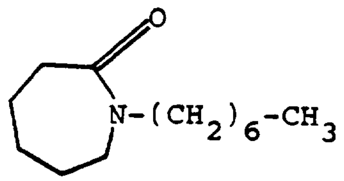

In a 1 liter 3-neck flask equipped with a water condenser, an addition funnel and a mechanical stirrer is placed 10.2 g of 50% sodium hydride-mineral oil dispersion (5.1 g NaH, 0.2125 M) and 150 ml of petroleum ether. The suspension is momentarily stirred and then sodium hydride is allowed to settle. Most of the petroleum ether is pipetted out and 200 ml of dry toluene is added, after which a solution of 20 g (0.177 M) of 2-caprolactam-2-one in 100 ml of dry toluene is added. The mixture is refluxed for 1 hour and then cooled to room temperature. A solution of 38.8 g (0.25 M) of 1-bromoheptane in 100 ml of dry toluene is added dropwise under stirring. Upon completion of the addition, the mixture is warmed to 80°-100° and the temperature was maintained for 4 hours. The reaction mixture is then heated to reflux for 18 hours to give 90% of a colorless product having a b.p. of 155°-158°C. at 0.5 mm Hg.

EXAMPLE XIX

Preparation of N-n-decylcaprolactam having the formula

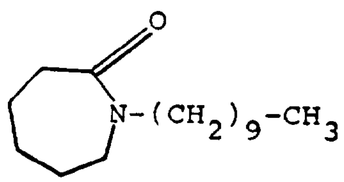

Following Example XVIII, 10.2 g of 50% sodium hydride-mineral oil dispersion (5.1 g NaH, 0.2125 M), 20 g (0.177 M) of azacycloheptan-2-one and 44.2 g (0.2 M) of I-bromodecane on 19 hr. reflux gives 38 g (84.7%) of product; b.p. 158°-163°/0.25-0.3 mm Hg.

EXAMPLE XX

Preparation of N-n-dodecylcaprolactam having the formula

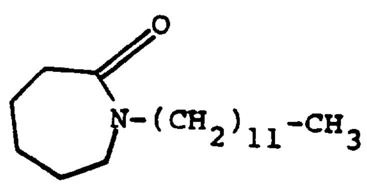

Following Example XVIII, 15.3 g of 50% sodium hydride-mineral oil dispersion (7.65 g NaH, 0.319 M), 30 g (0.266 M) of azacycloheptan-2-one and 66.1 g (0.265 M) of 1-bromododecane on 20 hours reflux gives 58.9 g (80%) of colorless product; b.p. 175°-180°/0.3 mm Hg.

EXAMPLE XXI

SURFACE TENSION

Surface tension measurements in triplicate were made for each of the N-alkylpyrrolidone species listed below in Table 1, using a Fisher Surface Tensiomat (Model #21, Der Nouy Ring Tensionometer). Each experiment was carried out as follows.

Distilled water solutions at concentrations noted in Table 1 were prepared for each of the following surfactants in 100 ml glass flasks. The solutions were stirred for about 15 minutes until homogeneous solutions were obtained. The surface tensions of these solutions were then measured.

The averaged results of the above tests are reported in Table 1.

TABLE 1

| Surface Tension of Aqueous Solutions at 25°C. (dynes/cm) | | | | | |
|---|---|---|---|---|---|
| | % Solution in Distilled Water | | | | |
| Product | 1.0% | 0.1% | 0.01% | 0.001% | 0.0001% |
| Octyl pyrrolidone | -- | 30.4 | 54.25 | 68.4 | -- |
| Decyl pyrrolidone | 27.6 | 27.5 | 27.8 | 51.0 | -- |
| Dodecyl pyrrolidone | -- | 26.7 | 27.6 | 29.9 | 55.4 |
| Tetradecyl pyrrolidone | -- | 26.4 | 26.5 | 30.4 | 47.5 |

Surface tension data indicates that these products have strong surface activity, i.e., lowering the surface tension of water.

From the surface tension data, the surface excess concentration $\Gamma$ (moles/cm$^2$), area/molecule ($a_m$Å$^2$)* at the interface and efficiency of adsorption, ($pC_{20}$)** at the solution/air interface were computed by use of appropriate Gibb's Adsorption Equation:

$$\Gamma = \frac{-d\gamma}{d \log c_T} / 2.303\ RT$$

where

$\Gamma$ = surface excess concentration (moles/cm$^2$)
$d\gamma$ = change in surface or interfacial tension of the solvent
R = 8.31 x 10 ergs mol$^{-1}$
c = Molar concentration of solution
T = Absolute Temperature

EXAMPLE XXII

The critical micelle concentration (CMC), the surface concentration at the liquid-air interface ($\Gamma$), the area of the test molecule at the interface (am) and the effectiveness of adsorption at the interface (pC-20) were determined on the above surface tension data reported in Example XXI, Table 1. These properties are reported in Table 2 below.

* Absorption Å$^2$
** The negative logarithm of the surfactant concentration required to lower surface tension by 20 dynes/cm.

TABLE 2

SURFACE ACTIVITY PARAMETERS CALCULATED FROM SURFACE TENSION MEASUREMENTS

| Molecule | CMC ($ml^{-1}$) | $\Gamma m (mcm^{-2} x 10^{10})$ | am $(Å)^2$ | pC-20 |
|---|---|---|---|---|
| N-n-octyl pyrrilidone | - | 4.2 | 39.5 | 3.20 |
| N-n-decyl pyrrolidone | $4.4 \times 10^{-4}$ | 4.6 | 36.1 | 4.51 |
| N-n-dodecyl pyrrolidone | $4.7 \times 10^{-5}$ | 4.5 | 36.9 | 5.27 |
| N-n-tetradecyl pyrrolidone | $5.7 \times 10^{-5}$ | 3.1 | 53.6 | 6.25 |
| lauryl dimethyl amine oxide* | $2.1 \times 10^{-3}$* | 2.8 | 59.3 | 5.23 |

* Literature value $\Gamma m = 3.5 \times 10^{-10}$ m $cm^{-1}$, am= $47Å^2$ (SURFACTANTS AND INTERFACIAL PHENOMENA by Rosen, page 68).

More specifically, the CMC of the N-(n alkyl) pyrrolidones tested show a minimum ($4.7 \times 10^{-5}$) at the $C_{12}$ chain length. Using the empirical equation for n-lauryl ($C_{12}$) alcohol ethoxylate,

$$\log C_{CMC} = A^1 + B^1 n$$

where $A^1 = 4.4$, $B^1 = 0.046$, and n = number of ethylene oxide at 23°C., the pyrrolidone ring would be expected to behave as 2 ethylene oxide units. However, the pyrrolidone ring is actually producing results equivalent to about 6

ethylene oxide units, i.e. at 25°C., n-$C_{12}H_{25}$ (ethylene oxide)$_4$ OH (CMC = 4 x $10^{-5}$) and n-$C_{12}H_{25}$ (ethylene oxide)$_7$OH (CMC = 5 x $10^{-5}$). The hydrophilic-lipophilic balance (HLB) of N-n-dodecyl pyrrolidone, based on the assumption that the pyrrolidone ring simulates 2-6 ethylene oxide units is 6.5 - 11 using the equation % ethylene oxide/5 - HLB. The low apparent HLB suggests the material would be a good water-in-oil emulsifier, e.g. in skin care products.

The surface concentration $\Gamma$, in moles per $cm^2$ indicates the maximum surface concentration of surfactant, i.e. at equilibrium above the CMC, and is obtained by an estimation of a constant d $\gamma$ /dc* from the surface tension vs. concentration. The N-(n alkyl) pyrrolidones obtain a maximum Tm (4.6 x $10^{-10}$m $cm^{-2}$) at N-$C_{10}H_{21}$ chain length. In general, the Tm for all of the N-n alkyl pyrrolidones is very high in comparison to all, but the less soluble alcohol ethoxylates, i. e. N-$C_{12}H_{25}$ (ethylene oxide)$_4$OH (Tm = 3.8 x $10^{-10}$m $cm^{-2}$) n-$C_{16}H_{33}$ (ethylene oxide)$_6$OH (Tm = 4.4 x $10^{-10}$m $cm^{-2}$). The mono disperse nature of the hydrophobe and hydrophile is believed to contribute to high surface concentrations by maximizing enthropy of packing.

As the interface becomes saturated with surfactant molecules, one would expect that within a homologous series, the longer hydrophobic chains would sterically hinder more surfactant molecules from positioning themselves at the interface. The decrease in Tm of the $C_8H_{17}$ vs. the $C_{10}H_{23}$ chain is attributed to the hydrophile/hydrophile interactions of the n-octyl-2-pyrrolidone. Above the $C_{10}H_{23}$ alkyl chain length, normal steric hinderance of the hydrophobes determines Tm.

The area per molecule (Å$^2$) is relative to the Tm, in a comparison of a homologous series. Generally, the area per molecule increases with increasing chain length of the N-(n alkyl) pyrrolidones; however, the larger am of the n-octyl pyrrolidone is attributed to an attraction of pyrrolidone moieties not balanced by the alkyl chain attractions. The low am of the N-(n alkyl) pyrrolidones approaches the theoretical size (20Å$^2$) for an aliphatic chain oriented perpendicular to the interface.

The negative logorithm of the surfactant concentration required to lower the surface tension by 20 dynes/$cm^{-1}$ can be used as a measure of the efficiency of adsorption since $\Gamma$ is approaching the maximum value. The N-(n alkyl) pyrrolidones actually show a progressively higher efficiency of adsorption as the alkyl chain is increased.

EXAMPLE XXIII

SOLUBILITY OF N-ALKYL PYRROLIDONES

Separate 10% by weight solutions of N-alkyl-2-pyrrolidone in the following liquids were made up in 50 ml glass beakers and the solubilities at about 25°C. were noted and reported in following Table 9.

* d $\gamma$/dc = the change in surface tension with respect to the change in surfactant concentration.

TABLE 9

10% PRODUCT SOLUBILITY AT $25^{o}C \pm 1^{o}C$

| SOLVENT | N-octyl-2-pyrrolidone | N-decyl-2-pyrrolidone | N-dodecyl-2-pyrrolidone | N-tetradecyl-2-pyrrolidone |
|---|---|---|---|---|
| Water | I | PS* | S* | S* |
| Acetone | S | S | S | S |
| Ethanol | S | S | S | S |
| Xylene | S | S | S | S |
| Heptane | S | S | S | S |
| Paraffin Oil | S | S | PS | S |
| Stoddard Solvent (1) | S** | S | PS | S |
| Perchloroethylene | S | S | S | S |

I= insoluble; PS= partially soluble (cloudy translucent); S= soluble, clear

(1) US Bureau of Standards specification- a clear, water white petroleum distillate

*Soluble with slight haze

**Very slight precipitation

The data indicates that alkyl pyrrolidones are soluble in various types of solvents. Although octyl pyrrolidone is insoluble at room temperature in water, decyl pyrrolidone is slightly soluble and dodecyl pyrrolidone and tetradecyl pyrrolidone are completely soluble in water at 10% concentration. However, dodecyl pyrrolidone and tetradecyl pyrrolidone exhibit iridescence phenomena at the 1% to 2% level in aqueous media (violet color at 2% and greenish at about

1%) which was destroyed in the presence of small amount of electrolyte.

It was also observed that 10% aqueous solution of tetradecyl pyrrolidone and dodecyl pyrrolidone become cloudy at about 33-34°C. and 31-32°C. respectively exhibiting cloud point phenonomena.

The aqueous solubility of the dodecyl and tetradecyl products is interesting, i.e. below 1% (wt. %) solute, the solutions are clear. Between 1 and 2% the solutions exhibit a scattering pattern (breaks light into its visible spectrum) for visible light indicating a regular geometric pattern which could be regular crystalline spacing (lamellar type) or a monodisperse liquid crystal particle. Above 2% the solutions again become clear, suggesting a self dissolving effect (loss of regular geometry). This effect once again demonstrates the purity of these compounds. It is postulated that blends of n-alkyl chains would decrease the ability of these materials to form lamellar type structures and increase the aqueous solubility of the less soluble compounds ($C_8$ and $C_{10}$). Also it has been observed that low levels of electrolyte eliminate the "prism effects" shown by the 1-2% solutions of the $C_{10}$-$C_{12}$ compounds.

EXAMPLE XXIV

The viscosities in cps of the following surfactants at various concentration in distilled water were measured at about 25°C. using a Brookfield Viscometer (LVT Model). The results of these tests are reported in following Table 10.

TABLE 10

VISCOSITY (CPS) AT VARIOUS WEIGHT % WATER CONCENTRATIONS

| PRODUCT | 100% | 10% | 8% | 6% | 4% | 2% |
|---|---|---|---|---|---|---|
| N-octyl-2-pyrrolidone | 8.0 | - | - | - | - | - |
| N-decyl-2-pyrrolidone | 12.0 | - | - | - | - | - |
| N-dodecyl-2-pyrrolidone | 17.0 | 20.0 | 16.0 | 12.5 | 7.5 | 6.5 |
| N-tetradecyl-2-pyrrolidone | 20.0 | 145.0 | 100.0 | 96.0 | 66.0 | 24.5 |
| Ammonyx LO | - | 7.0 | - | - | - | 4.0 |
| Ammonyx MO | - | 52.5 | - | - | - | 7.0 |
| Control (distilled water) | 1.0 | - | - | - | - | - |

The above viscosity of alkyl pyrrolidones and their aqueous solutions were obtained usinq a #2 spindle at a speed of 30 rpm, and a #2 spindle at a speed of 60 rpm respectively.

Data indicates that viscosity of the (100% active) alkyl pyrrolidone increased as the carbon chain length of alkyl group increased.

Both tetradecyl and dodecyl pyrrolidone exhibit a significant thickening increase. The higher viscosities of the N-n alkyl pyrrolidones vs. dimethyl fatty amine oxides of similar chain length indicate that the micelles of the N-n alkyl pyrrolidones are lamellar or rod shaped as compared to spherical types for the amine oxides.

EXAMPLE XXV

VISCOSITY BUILDING

Several samples to be tested were prepared in 1500 ml glass beakers by mixing 47.9 wt. % of deionized water at 60°C. with 50 wt. % surfactant and 0.6 wt. % of the viscosity builder (100% active) to be tested while agitating constantly with an electric stirrer. The resulting solution was allowed to cool to 40°C. whereupon 0.05 wt. % of Kathon CG preservative was added with stirring and the solution allowed to cool to room temperature.

A control solution was prepared for each surfactant used in the same manner except that viscosity builder was omitted from the control formulations and 49.95 wt. % of deionized water was employed.

The viscosity was measured in cps for each of the above solutions by adding 20% NaCl solution from a glass pipette in 2 ml increments until the peak viscosity break point was reached. Anhydrous sodium chloride was used when requirements exceeded 4% NaCl by weight. The results of these tests which report the viscosity at the break point for each test solution and for each control are reported in the following Table 11.

TABLE 11

| | VISCOSITY (CPS) |
|---|---|
| **SURFACTANT= Na lauryl sulfate** | |
| Viscosity Builder | |
| N-dodecyl-2-pyrrolidone | 12,285 |
| coconut diethanol amide | 13,000 |
| lauryl dimethylamine oxide | 10,140 |
| cocamidopropyl betaine | 17,850 |
| cocamidopropyl hydroxy sultaine | 12,425 |
| Control | 1,850 |
| **SURFACTANT= Na laureth sulfate (3 moles ethylene oxide)** | |
| Viscosity Builder | |
| N-dodecyl-2-pyrrolidone | 40,000 |
| coconut diethanol amide | 36,820 |
| lauryl dimethylene oxide | 31,590 |
| cocamidopropyl betaine | 55,680 |
| cocamidopropyl hydroxy sultaine | 54,540 |
| Control | 30,000 |
| **SURFACTANT= Ammonium lauryl sulfate** | |
| Viscosity Builder | |
| N-dodecyl-2-pyrrolidone | 40,000 |
| coconut diethanol amide | 33,200 |
| lauryl dimethylamine oxide | 37,270 |
| cocamidopropyl betaine | 54,590 |
| cocamidopropyl hydroxy sultaine | 28,180 |
| Control | 6,360 |

EXAMPLE XXVI

Protonation of N-n Alkyl Pyrrolidones to Form Cations

The cation formed by protonation of N-dodecyl pyrrolidone, i.e.

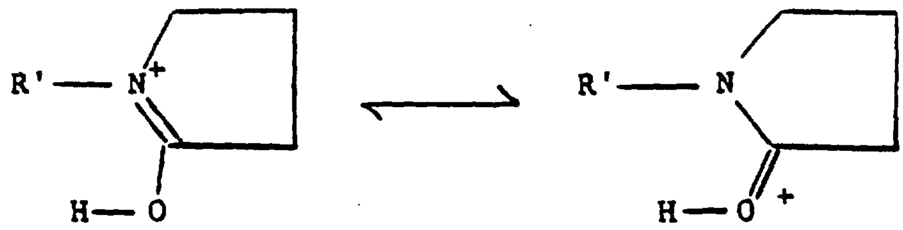

was precipitated from acid solution using a large anionic surfactant molecule, linear dodecyl benzene sulfonate.

Both the $C_8$ and $C_{10}$ alkyl pyrrolidones which are rather insoluble in water at pH 7, are readily solubilized in acid solution. Ten percent dispersions of the $C_8$ and $C_{10}$ pyrrolidones were titrated to a clear solution using HCl. The results are shown in the following table, Table 12.

TABLE 12

MOLAR RATIO OF HCl TO ALKYL PYRROLIDONE REQUIRED FOR SOLUBILIZATION

| N-n-alkyl group | Grams of Pyrrolidone (10% Dispersion) | HCl (ml.) 36.7% | Mole Ratio HCl/Pyrrolidone | Solution pH |
|---|---|---|---|---|
| octyl | 20.0 | 5.2 | 6.2 | 0.22 |
| decyl | 20.0 | 9.7 | 12.8 | $<0.1$ |

To obtain a rough figure for the protonation equilibrium constant, the equilibrium [octyl pyrrolidone] was taken at $5 \times 10^{-3}$m. Additionally, the specific gravity of the 10% dispersion was taken at 1.0 g/cc. calculation indicates that $K = 1.3 \times 10^{2}$ for the octyl pyrrolidone forming a cation, i.e. [octyl pyrrolidone] + $[H^{+}] \rightleftarrows$ [protonated pyrrolidone $]^{+}$.

Solubility of the present surfactants in highly alkaline solutions is limited and the pyrrolidone ring is not hydrolyzed in such environments. However the solubility in acid is very high. Data indicate that the present N-alkyl-2-pyrrolidones are much less prone to acid hydrolysis than N-methyl pyrrolidone. Mixtures (50/50) of the N-n alkyl pyrrolidones with 36.7% HCl were made. The $C_8$ and $C_{10}$ alkyl pyrrolidones were clear, viscous liquids at 25°C., while the $C_{12}$ alkyl/HCl

mix was a homogeneous, transparent gel. The $C_{14}$/HCl (50/50) mixture became a non-homogeneous solid at room temperature.

EXAMPLE XXVII

Anti-Corrosion Testing

The 9 N-alkyl-2-pyrrolidone products listed in the following table were tested in hydrochloric acid and contacted with oil well casing material to determine their efficacy as corrosion inhibitors.

Separate solutions each containing 0.4 g of the test compound in 100 mls of 15% active hydrochloric acid, were made up.

Steel Haliburton N-80 coupons used in oil well drilling were soaked in a 20% soap solution (Alkanox) for 20 minutes, then brush scrubbed with yellow soap and rinsed with water and then with acetone. The coupons were dried and weighed.

After weighing, a coupon was placed in each of the beakers containing a test compound 20% HCl solution and warmed to 80°C, for 16 hours, after which the coupons were removed, rinsed, brush scrubbed with soap solution, dried and reweighed. The loss in weight of the coupon is recorded in following Table 13.

TABLE 13

| Steel Casing Corrosion Inhibitor Evaluation | Weight Loss (%) |
|---|---|
| Propargyl alcohol | 0.21 |
| N-decyl pyrrolidone (DP) | 4.10 |
| N-octyl pyrrolidone (OP) | 4.20 |
| N-dodecyl pyrrolidone (DDP) | 5.95 |
| N-cyclohexyl pyrrolidone (CHP) | 10.97 |
| N-methoxyethyl pyrrolidone (MEP) | 14.16 |
| N-methyl pyrrolidone (NMP) | 14.55 |
| N-ethyl pyrrolidone (NEP) | 14.56 |
| N-tetradecyl pyrrolidone (TDP) | 14.82 |
| N-hydroxyethyl pyrrolidone (HEP) | 15.57 |
| No inhibitor | 27.2 |

Although propargyl alcohol showed the lowest weight loss, the present N-alkyl pyrrolidones present a viable alternative and are safer chemicals.

The above experiment was repeated except that the coupons were immersed in a 5% HCl solution for 16 hours. From this data, a corrosion inhibiting curve was plotted for the above inhibitors as shown in Figure 1 wherein % weight loss is plotted against the weight of the alkyl group. 2-Pyrrolidone (2P) was used for purposes of comparison by immersing a coupon for 16 hours at 80°C. in a 5% HCl solution containing 0.4 g of 2P. Interpolation of this curve indicates that N-nonyl-2-pyrrolidone is at least as effective as the N-octyl or the N-decyl species. N-tetradecyl pyrrolidone is a suitable corrosion inhibitor for weaker acids.

It is also found that the octyl, decyl, and dodecyl pyrrolidones are better inhibitors than cationic quaternary ammonium chloride in isopropanol such as the commercial corrosion inhibitor Katapone VV-328, which gave a 16% weight loss of the coupons in 15% HCl. Also this dark-colored quaternary ammonium chloride which is shipped as a 75% solution in isopropanol compares unfavorably with the present alkyl pyrrolidones which are water clear, 100% active and water soluble.

EXAMPLE XXVIII

The density at 25°C., heat of vaporization in kilocalorie per mole and solubility parameter was determined for the $C_8$, $C_{10}$, $C_{12}$ and $C_{14}$ alkyl pyrrolidone species of the invention. These parameters are reported in following Table 14.

TABLE 14

| PRODUCT | $d_{25^oC}$ | Heat of Vaporizn. k cal/mole | Solubility Parameter (δ) |
|---|---|---|---|
| N-n-octyl-2-pyrrolidone | 0.920 | 15.0 | 8.2 |
| N-n-decyl-2-pyrrolidone | 0.911 | 18.4 | 8.5 |
| N-n-dodecyl-2-pyrrolidone | 0.903 | 18.0 | 7.9 |
| N-n-tetradecyl-2-pyrrolidone | 0.896 | 19.7 | 7.8 |

Example XXIX

Some representative formulations which suitably employ the products of this invention are presented below, although these are by no means limiting to the scope of suitable mixtures. The following formulations and compositions are prepared by conventional methods and require no detailed description. Generally the components are mixed at between about room temperature and about 100°C. under ambient pressure until a uniform composition is obtained.

FF. Herbicidal and Fungicidal Adjuvant Formulation

The following formulation has been specially developed for triazine herbicides in post emergent applications in order to increase the application rate without reducing weed control and, at the same time, reducing harmful residues, thus allowing quick crop rotation.

The present N-alkyl lactams can be built into a suspension concentrate without detracting from the physical stability of the product.

A concentrate formulation

| | |
|---|---|
| Atrazine (2-chloro-4-ethylamino-6-isopropyl-amino,-1,3,5-triazine) | 250 grams |
| N-n-octyl-2-pyrrolidone | 328 grams |
| $H_2O$ | to 1 liter |

GG. Emulsifiable Concentrate Formulations for Agricultural Chemicals

(i) The following formulations (1-5) describe herbicidal 2,4-D (isopropyl ester of 2,4-dichlorophenoxy acetic acid) emulsifiable concentrates in various solvents which contain a 50/50 wt % mixture of N-dodecyl-2-pyrrolidone and Emulphor EL-620 and which, when added to water, produce stable emulsions.

| # | herbicide % by Wt. | Surfactant Blend % by Wt. | Xylene % by Wt. | Kerosene % by Wt. | Velsicol* AR-50 % by Wt. | Shell** F-407-R % by Wt. |
|---|---|---|---|---|---|---|
| 1 | 39 | 5 | - | 56 | - | 1 |
| 2 | 44 | 5 | 51 | - | - | - |
| 3 | 44 | 5 | - | - | 51 | - |
| 4 | 40 | 5 | - | - | 21 | - |
| 5 | 47 | 5 | - | - | - | 48 |

The same formulations can be used for the butyl and other alkyl esters of 2,4-D. The addition of the pyrrolidone mixture in the above formulation controls viscosity, provides a stable emulsion and better distribution of the formulation on the vegetation.

(ii) The following formulations (1-7) describe suitable insecticidal (chlordane) emulsifiable concentrates containing a 10/40 wt. % blend of N-n-octyl-2-pyrrolidone and Emulphor EL-620 optionally combined with varying amounts of Igepal CO-630. The Igepal containing blends when added to water produce fast breaking emulsions; whereas those omitting Igepal are stable.

| # | Chlordane* wt. % | Igepal CO-630 wt. % | Pyrrolidone/Emulphor Blend wt. % | Butyl Cellosolve wt. % | Kerosene wt. % |
|---|---|---|---|---|---|
| 1 | 50 | - | 35 | - | - |
| 2 | 50 | - | 35 | 15 | - |
| 3 | 46 | 2 | - | - | 46 |
| 4 | 46 | 2.5 | - | - | 49 |
| 5 | 75 | - | 5 | - | 20 |
| 6 | 75 | 10 | - | - | 15 |
| 7 | 46 | 9 | - | - | 45 |

* 1,2,4,5,6,7,8,8-Octachloro-2,3,3a,4,7,7a-hexahydro-4,7-methanoindane

(iii) The following formulations (1-4) describe suitable insecticidal (toxaphene) emulsifiable concentrates containing a 20/20/40 blend of N-n-decyl-2-pyrrolidone, N-n-dodecyl-2-pyrrolidone and Emulphor FL-620, optionally combined with varying amounts of Igepal CO-530. The Igepal containing blends, when added to water provide fast breaking emulsions; whereas those which omit Igepal provide stable emulsions.

| # | Toxaphene* wt. % | Igepal CO-530 wt. % | Surfactant Blend wt. % | Kerosene wt. % | Butyl Cellosolve wt. % |
|---|---|---|---|---|---|
| 1 | 50 | 15 | 35 | - | - |
| 2 | 50 | - | 35 | - | 15 |
| 3 | 45 | 8 | - | 47 | - |
| 4 | 45 | 4 | - | 50 | - |

* chlorinated camphene

The pyrrolidone in the toxaphene formulation provides the same promotional effect as noted for chlordane.

HH. Wettable Powder Formulations

(i)

| | % by Wt. |
|---|---|
| Isopropylphenyl carbamate | 50.00 |
| Hi-Sil (hydrated amorphorous silica) | 46.00 |
| Marasperse N (sodium lignosulfonate) | 2.00 |
| N-n-octyl-2-pyrrolidone | 2.00 |

Above formulation has excellent suspension and dispersion in hard and soft water.

(ii)

| | % by Wt. |
|---|---|
| Chlordane | 40.00 |
| Attaclay (attapulgite) | 55.00 |
| Blancol | 3.00 |
| N-n-octyl-2-pyrrolidone | 2.00 |

(iii)

| | % by Wt. |
|---|---|
| Chlordane | 40.00 |
| Attaclay | 56.50 |
| Marasperse N | 2.00 |
| Igepon T 77 | 0.50 |
| N-n-dodecyl-2-pyrrolidone | 1.00 |

(iv)

| | % by Wt. |
|---|---|
| Toxaphene | 40.00 |
| Attaclay | 56.00 |
| Daxad 27 (Na salt of a polymerized substituted benzoid alkyl sulfonic acid) | 3.00 |
| N-n-octyl-2-pyrrolidone | 1.00 |

(v)

| | % by Wt. |
|---|---|
| Toxaphene | 40.00 |
| Attaclay | 55.00 |
| Blancol | 4.00 |
| N-n-octyl-2-pyrrolidone | 1.00 |

(vi)

| | % by Wt. |
|---|---|
| Toxaphene | 40.00 |
| Attaclay | 55.00 |
| Marasperse N | 4.00 |
| N-n-octyl-2-pyrrolidone/N-n-dodecyl-2-pyrrolidone 75/25 mixture | 1.00 |

(vii)

| | % by Wt. |
|---|---|
| Aldrin (1,2,3,4,10,10-hexachloro-1,4,4a, 5,8,8a-hexahydro-exo-1,4-endo-5,8-dimethano-naphthalene | 25.00 |
| Attaclay | 71.50 |
| Blancol | 2.00 |
| Igepon T-77 | 0.50 |
| N-n-octyl-2-pyrrolidone | 1.00 |

(viii)

| | % by Wt. |
|---|---|
| Aldrin | 50.00 |
| Attaclay | 45.00 |
| Marasperse N | 3.00 |
| N-n-decyl-2-pyrrolidone | 2.00 |

(ix)

| | % by Wt. |
|---|---|
| Aldrin | 75.00 |
| Hi-Sil | 20.00 |
| Blancol | 3.00 |
| N-n-dodecyl-2-pyrrolidone | 2.00 |

(x)

| | % by Wt. |
|---|---|
| Dieldrin (Hexachloro-epoxy-octahydro-endo, exo-dimethanonaphthalene) | 25.00 |
| Attaclay | 71.50 |
| Blancol | 2.00 |
| Igepon T-77 | 0.50 |
| N-n-octyl-2-pyrrolidone | 1.00 |

(xi)

| | % by Wt. |
|---|---|
| Dieldrin | 50.00 |
| Attaclay | 45.00 |
| Blancol | 3.00 |
| N-n-dodecyl-2-pyrrolidone | 2.00 |

The above formulations are milled on a fly cutter mill at 20,000 rpm for 1 minute at room temperature and provide concentrates having a wetting time less than 30 seconds.

II. Fungicidal Wettable Powder Formulation

| | % by Wt. |
|---|---|
| Phenyl Mercuric Acetate | 90.00 |
| N-n-octyl-2-pyrrolidone | 1.00 |

The formulation provides a free flowing, non-bleeding powder; however only 0.1 to about 0.5% by weigh of N-n-octyl-2-pyrrolidone is required to impart good wettability to this fungicidal powder.

## Claims

**1.** A composition comprising

(i) a N-hydrocarbon substituted lactam surfactant having the formula

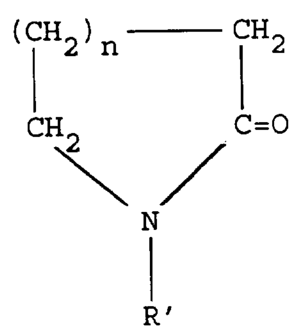

wherein n is an integer having a value of from 1 to 3 and R' is a hydrophobic radical consisting of a linear, branched chain or cyclic alkyl radical containing from 7 to 22 carbon atoms; a naphthyl or alkyl substituted naphthyl radical containing from 10 to 26 carbon atoms or an alkylphenyl or phenylalkyl radical containing from 9 to 26 carbon atoms; which lactams are capable of forming micelles in neutral, basic or acidic aqueous media or have a critical micelle concentration of between about 1 x $10^{-3}$ and about 5 x $10^{-5}$ moles per liter, and
(ii) at least one water-insoluble substance which is other than a said lactam surfactant, and is selected from insecticides, herbicides, fungicides, pesticides, plant growth regulators and annelidicides;
which composition is in the form of a liquid concentrate or a wettable powder and forms an emulsion or suspension of the substance upon dilution with water.

2. A composition according to claim 1 wherein R' is alkyl.

3. A composition according to claim 2 wherein n is 1 and R' is alkyl having from 8 to 18 carbon atoms.

4. A composition according to any one of the preceding claims wherein the water-insoluble substance is toxic and the substituted lactam surfactant lowers the toxicity thereof.

5. A composition according to any one of the preceding claims which is a liquid concentrate and forms an emulsion of said water-insoluble substance upon dilution with water, said composition comprising at least one amphoteric, anionic, nonionic or cationic surfactant in addition to said lactam surfactant.

6. A composition according to claim 5 wherein the weight ratio of said lactam surfactant and additional surfactant lies in a range from 1:10 to 1:0.8.

7. A process of applying a water-insoluble substance to land or crops comprising the steps of diluting a composition containing the substance with water, to form an aqueous emulsion or suspension of the substance, and then spraying the emulsion or suspension onto the crops or land, the composition comprising

(i) a N-hydrocarbon substituted lactam surfactant having the formula

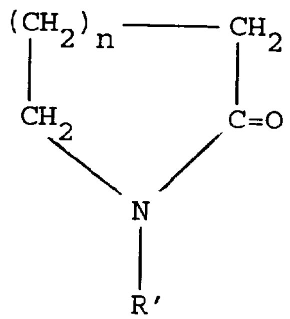

wherein n is an integer having a value of from 1 to 3 and R' is a hydrophobic radical consisting of a linear, branched chain or cyclic alkyl radical containing from 7 to 22 carbon atoms; a naphthyl or alkyl substituted naphthyl radical containing from 10 to 26 carbon atoms or an alkylphenyl or phenylalkyl radical containing from 9 to 26 carbon atoms; which lactams are capable of forming micelles in neutral, basic or acidic aqueous media or have a critical micelle concentration of between about 1 x $10^{-3}$ and about 5 x $10^{-5}$ moles per liter, and
(ii) at least one water-insoluble substance which is other than a said lactam surfactant.

8. A process according to claim 7 wherein R' is alkyl.

9. A process according to claim 8 wherein n is 1 and R' is alkyl having from 8 to 18 carbon atoms.

10. A process according to any one of claims 7 to 9 wherein the composition is a liquid concentrate and forms an emulsion of said water-insoluble substance upon dilution with water, said composition comprising at least one amphoteric, anionic, nonionic or cationic surfactant in addition to said lactam surfactant.

11. Use of an N-hydrocarbon substituted lactam surfactant having the formula

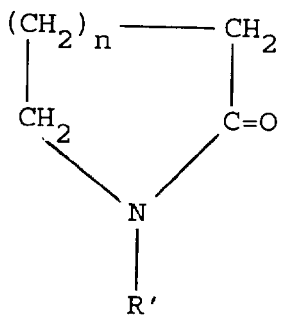

wherein n is an integer having a value of from 1 to 3 and R' is a hydrophobic radical consisting of a linear, branched chain or cyclic alkyl radical containing from 7 to 22 carbon atoms; a naphthyl or alkyl substituted naphthyl radical containing from 10 to 26 carbon atoms or an alkylphenyl or phenylalkyl radical containing from 9 to 26 carbon atoms; which lactams are capable of forming micelles in neutral, basic or acidic aqueous media or have a critical micelle concentration of between about 1 x $10^{-3}$ and about 5 x $10^{-5}$ moles per liter; to form a concentrate containing at least one water-insoluble substance, which concentrate is suitable for dilution with water to yield an aqueous suspension or emulsion of the substance for spraying onto crops or land.

**12.** Use according to claim 11 wherein R' is alkyl.

**13.** Use according to claim 12 wherein n is 1 and R' is alkyl having from 8 to 18 carbon atoms.

**14.** Use according to any one of claims 11 to 13 wherein the composition is a liquid concentrate and forms an emulsion of said water-insoluble substance upon dilution with water, said composition comprising at least one amphoteric, anionic, nonionic or cationic surfactant in addition to said lactam surfactant.

**Patentansprüche**

**1.** Zusammensetzung, umfassend

(i) ein mit einem Kohlenwasserstoff-Rest N-substituiertes Lactam-Tensid der Formel

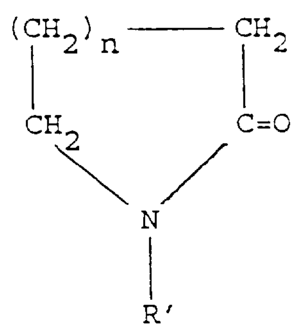

worin n eine ganze Zahl mit einem Wert von 1 bis 3 ist und R' ein hydrophober Rest ist, bestehend aus einem linearen, verzweigten oder zyklischen Alkylrest mit 7 bis 22 Kohlenstoffatomen, einem Naphthyl- oder alkylsubstituierten Naphthylrest mit 10 bis 26 Kohlenstoffatomen oder einem Alkylphenyl- oder Phenylalkylrest mit 9 bis 26 Kohlenstoffatomen; welche Lactame in neutralen, basischen oder sauren wäßrigen Medien Micellen bilden können oder eine kritische Micellenkonzentration zwischen etwa 1 x $10^{-3}$ und etwa 5 x $10^{-5}$ Mol pro Liter aufweisen, und

(ii) zumindest eine wasserunlösliche Substanz, die kein Lactam-Tensid und aus Insektiziden, Herbiziden, Fungiziden, Pestiziden, Pflanzenwachstumsreglern und Annelidiziden ausgewählt ist;

welche Zusammensetzung in Form eines flüssigen Konzentrats oder eines benetzbaren Pulvers vorliegt und bei Verdünnung mit Wasser eine Emulsion oder Suspension der Substanz bildet.

**2.** Zusammensetzung nach Anspruch 1, worin R' ein Alkyl ist.

3. Zusammensetzung nach Anspruch 2, worin n = 1 und R' ein Alkyl mit 8 bis 18 Kohlenstoffatomen ist.

4. Zusammensetzung nach einem der vorhergehenden Ansprüche, worin die wasserunlösliche Substanz toxisch ist und das substituierte Lactam-Tensid deren Toxizität mindert.

5. Zusammensetzung nach einem der vorhergehenden Ansprüche, die ein flüssiges Konzentrat ist und bei Verdünnung mit Wasser eine Emulsion der wasserunlöslichen Substanz bildet, wobei die Zusammensetzung zusätzlich zum Lactam-Tensid zumindest ein amphoteres, anionisches, nichtionogenes oder kationisches Tensid umfaßt.

6. Zusammensetzung nach Anspruch 5, worin das Gewichtsverhältnis zwischen dem Lactam-Tensid und dem zusätzlichen Tensid im Bereich von 1:10 bis 1:0,8 liegt.

7. Verfahren zum Aufbringen einer wasserunlöslichen Substanz auf Erde oder auf Erntefrüchte, umfassend die Schritte des Verdünnens einer die Substanz enthaltenden Zusammensetzung mit Wasser, um eine wäßrige Emulsion oder Suspension der Substanz zu bilden, und des Spritzens der Emulsion oder Suspension auf die Erntefrüchte oder die Erde, wobei die Zusammensetzung folgendes umfaßt:

(i) ein mit einem Kohlenwasserstoff-Rest N-substituiertes Lactam-Tensid der Formel

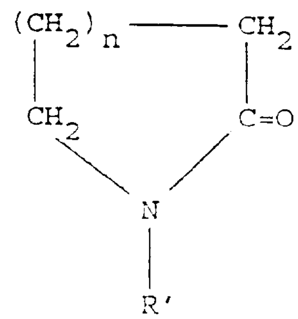

worin n eine ganze Zahl mit einem Wert von 1 bis 3 ist und R' ein hydrophober Rest ist, bestehend aus einem linearen, verzweigten oder zyklischen Alkylrest mit 7 bis 22 Kohlenstoffatomen, einem Naphthyl- oder alkylsubstituierten Naphthylrest mit 10 bis 26 Kohlenstoffatomen oder einem Alkylphenyl- oder Phenylalkylrest mit 9 bis 26 Kohlenstoffatomen; welche Lactame in neutralen, basischen oder sauren wäßrigen Medien Micellen bilden können oder eine kritische Micellenkonzentration zwischen etwa 1 x $10^{-3}$ und etwa 5 x $10^{-5}$ Mol pro Liter aufweisen, und

(ii) zumindest eine wasserunlösliche Substanz, die kein Lactam-Tensid ist.

8. Verfahren nach Anspruch 7, worin R' ein Alkyl ist.

9. Verfahren nach Anspruch 8, worin n = 1 und R' ein Alkyl mit 8 bis 18 Kohlenstoffatomen ist.

10. Verfahren nach einem der Ansprüche 7 bis 9, worin die Zusammensetzung ein flüssiges Konzentrat ist und beim Verdünnen mit Wasser eine Emulsion der wasserunlöslichen Substanz bildet, wobei die Zusammensetzung zusätzlich zum Lactam-Tensid zumindest ein amphoteres, anionisches, nichtionogenes oder kationisches Tensid umfaßt.

11. Verwendung eines mit einem Kohlenwasserstoff-Rest N-substituierten Lactam-Tensids der Formel

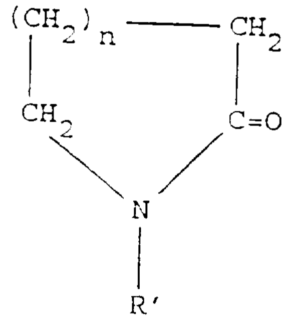

worin n eine ganze Zahl mit einem Wert von 1 bis 3 ist und R' ein hydrophober Rest ist, bestehend aus einem linearen, verzweigten oder zyklischen Alkylrest mit 7 bis 22 Kohlenstoffatomen besteht, einem Naphthyl- oder alkylsubstituierten Naphthylrest mit 10 bis 26 Kohlenstoffatomen oder einem Alkylphenyl- oder Phenylalkylrest mit 9 bis 26 Kohlenstoffatomen; welche Lactame in neutralen, basischen oder sauren wäßrigen Medien Micellen bilden können oder eine kritische Micellenkonzentration zwischen etwa 1 x $10^{-3}$ und etwa 5 x $10^{-5}$ Mol pro Liter aufweisen; zur Bildung eines Konzentrats, das zumindest eine wasserunlösliche Substanz enthält und sich zur Verdünnung mit Wasser eignet, um eine wäßrige Suspension oder Emulsion der Substanz zum Spritzen auf Erntefrüchte oder Erde zu ergeben.

**12.** Verwendung nach Anspruch 11, worin R' ein Alkyl ist.

**13.** Verwendung nach Anspruch 12, worin n = 1 und R' ein Alkyl mit 8 bis 18 Kohlenstoffatomen ist.

**14.** Verwendung nach einem der Ansprüche 11 bis 13, worin die Zusammensetzung ein flüssiges Konzentrat ist und beim Verdünnen mit Wasser eine Emulsion der wasserunlöslichen Substanz bildet, welche Zusammensetzung zusätzlich zum Lactam-Tensid zumindest ein amphoteres, anionisches, nichtionogenes oder kationisches Tensid umfaßt.

## Revendications

**1.** Composition comprenant

(i) une lactame tensioactive N-hydrocarbure substituée ayant pour formule

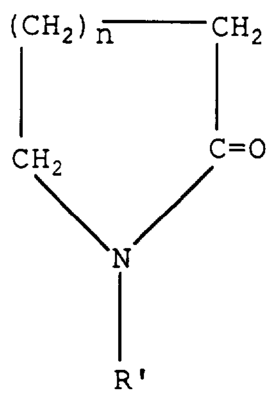

où n est un nombre entier ayant une valeur de 1 à 3 et R' est un radical hydrophobe consistant en un radical alkyle à chaîne linéaire, ramifiée ou cyclique contenant 7 à 22 atomes de carbone; un radical naphtyle naphtyle ou alkyle substitué contenant 10 à 26 atomes de carbone ou un radical alkylphényle ou phénylalkyle contenant 9 à 26 atomes de carbone; lesquelles lactames sont capables de former des micelles dans des milieux aqueux neutres, basiques ou acides ou ont une concentration critique en micelles, comprise entre environ 1 x $10^{-3}$ et environ 5 x $10^{-5}$ mole par litre, et

(ii) au moins une substance insoluble dans l'eau qui est autre que ladite lactame tensioactive et qui est sélectionnée parmi des insecticides, herbicides, fongicides, pesticides, régulateurs de la croissance de plantes et annélidicides;

laquelle composition est sous la forme d'un concentré liquide ou d'une poudre mouillable et forme une émulsion ou une suspension de la substance dans une dilution avec de l'eau.

**2.** Composition selon la revendication 1 où R' est alkyle.

**3.** Composition selon la revendication 2 où n est 1 et R' est alkyle ayant 8 à 18 atomes de carbone.

**4.** Composition selon l'une quelconque des revendications précédentes où la substance insoluble dans l'eau est toxique et la lactame tensioactive substituée abaisse sa toxicité.

**5.** Composition selon l'une quelconque des revendications précédentes qui est un concentré liquide qui forme une émulsion de ladite substance insoluble dans l'eau lors d'une émulsion avec de l'eau, ladite composition comprenant au moins un agent tensioactif amphotère, anionique, non ionique ou cationique, en addition à ladite lactame tensioactive.

**6.** Composition selon la revendication 5 où le rapport pondéral de ladite lactame tensioactive et de l'agent tensioactif additionnel est compris entre 1:10 et 1:0,8.

**7.** Procédé d'application d'une substance insoluble dans l'eau à de la terre ou des récoltes, comprenant les étapes de diluer une composition contenant la substance avec de l'eau pour former une émulsion ou suspension aqueuse de la substance puis de pulvériser l'émulsion ou suspension sur les récoltes ou la terre, la composition comprenant

(i) une lactame tensioactive N-hydrocarbure substituée ayant pour formule

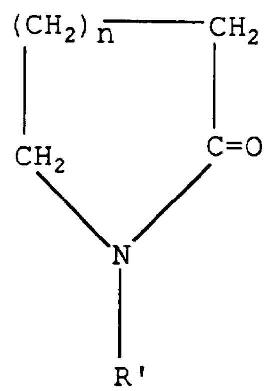

où n est un nombre entier ayant une valeur de 1 à 3 et R' est un radical hydrophobe consistant en un radical alkyle à chaîne linéaire, ramifiée ou cyclique contenant 7 à 22 atomes de carbone; un radical naphtyle naphtyle ou alkyle substitué contenant 10 à 26 atomes de carbone ou un radical alkylphényl ou phénylalkyle contenant 9 à 26 atomes de carbone; lesquelles lactames sont capables de former des micelles dans des milieux aqueux neutres, basiques ou acides ou ont une concentration critique en micelles comprise entre environ 1 x $10^{-3}$ et environ 5 x $10^{-5}$ mole par litre, et

(ii) au moins une substance insoluble dans l'eau qui est autre que ladite lactame tensioactive.

**8.** Procédé selon la revendication 7 où R' est alkyle.

**9.** Procédé selon la revendication 8 où n est 1 et R' est alkyle ayant 8 à 18 atomes de carbone.

**10.** Procédé selon l'une quelconque des revendications 7 à 9 où la composition est un concentré liquide et forme une émulsion de ladite substance insoluble dans l'eau lors d'une dilution avec de l'eau, ladite composition comprenant au moins un agent tensioactif amphotère, anionique, non ionique ou cationique, en plus de ladite lactame tensioactive.

**11.** Utilisation d'une lactame tensioactive N-hydrocarbure substituée ayant pour formule

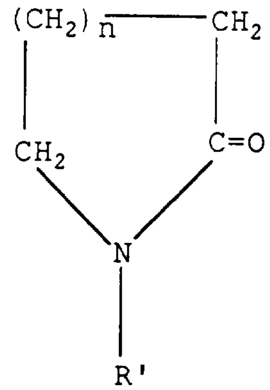

où n est un nombre entier ayant une valeur de 1 à 3 et R' est un radical hydrophobe consistant en un radical alkyle à chaîne linéaire, ramifiée ou cyclique contenant 7 à 22 atomes de carbone; un radical naphtyle naphtyle ou alkyle substitué contenant 10 à 26 atomes de carbone ou un radical alkylphényle ou phénylalkyle contenant 9 à 26 atomes de carbone; lesquelles lactames sont capables de former des micelles dans des milieux aqueux neutres, basiques ou acides ou ont une concentration critique en micelles, comprise entre environ 1 x $10^{-3}$ et environ 5 x $10^{-5}$ mole par litre; pour former un concentré contenant au moins une substance insoluble dans l'eau, lequel concentré est approprié à une dilution avec de l'eau pour donner une suspension ou émulsion aqueuse de la substance pour une pulvérisation sur des récoltes ou de la terre.

**12.** Utilisation selon la revendication 11 où R' est alkyle.

**13.** Utilisation selon la revendication 12 où n est 1 et R' est alkyle ayant 8 à 18 atomes de carbone.

**14.** Utilisation selon l'une quelconque des revendications 11 à 13 où la composition est un concentré liquide et forme une émulsion de ladite substance insoluble dans l'eau lors d'une dilution avec de l'eau, ladite composition comprenant au moins un agent tensioactif amphotère, anionique, non ionique ou cationique en plus de ladite lactame tensioactive.

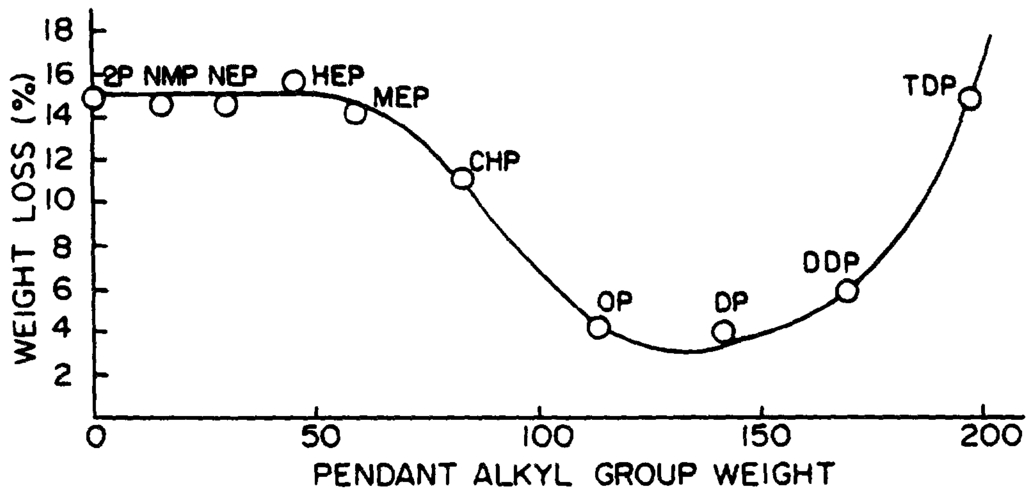
WEIGHT LOSS (%)
18
16
14
12
10
8
6
4
2
0
50
100
150
200
PENDANT ALKYL GROUP WEIGHT
2P
NMP
NEP
HEP
MEP
CHP
OP
DP
DDP
TDP

FIG. 1